# FASCICULE DE BREVET EUROPEEN

(11) **EP 2 445 874 B1**
(45) Date de publication et mention de la délivrance du brevet: **29.11.2017**
(21) Numéro de dépôt: 10726102.6
(22) Date de dépôt: 24.06.2010
(51) Int. Cl.: C07D 207/20, C07D 211/70

(54) **METHODE DE SYNTHESE DE LA 2-ACETYL-1-PYRROLINE ET DE SON PRECURSEUR STABLE, MARQUE ISOTOPIQUEMENT OU NON**
VERFAHREN FÜR DIE SYNTHESE VON 2-ACETYL-1-PYRROLIN UND SEINEM STABILEN VORLÄUFER MIT OPTIONALER ISOTOPENMARKIERUNG
PROCESS OF PREPARATION OF 2-ACETYL-1-PYRROLINE AND A STABLE PRECURSOR THEREOF, ISOTOPICALLY LABELLED OR NOT

(30) Priorité: 24.06.2009 FR 0954319
(43) Date de publication de la demande: 02.05.2012
(73) Titulaire: Centre National de la Recherche Scientifique (C.N.R.S.), 75016 Paris (FR); Université Montpellier 2 Sciences et Techniques, 34000 Montpellier (FR)
(72) Inventeur: MORERE, Alain, F-34270 Les Matelles (FR); MENUT, Chantal, F-34730 Saint Vincent De Barbeyrargues (FR); GUNATA, Yusuf Ziya, F-34070 Montpellier (FR); AGREBI, Abdelhamid, Tunis 2070 (TN)
(74) Mandataire: Regimbeau
(86) Numéro de dépôt international: PCT/EP2010/059007
(87) Numéro de publication internationale: WO 2010/149744

(56) Documents cités:
- EP-A- 0 436 481
- DE-A1- 4 217 395
- US-B1- 6 723 856
- FUGANTI ET AL: "A general method for the synthesis of the most powerful naturally occurring Maillard flavors" TETRAHEDRON, ELSEVIER SCIENCE PUBLISHERS, AMSTERDAM, NL, vol. 63, no. 22, 26 avril 2007 (2007-04-26), pages 4762-4767, XP022047408 ISSN: 0040-4020
- ADAMS AN ET AL: "Chemistry of 2-acetyl-1-pyrroline, 6-acetyl-1,2,3,4-tetrahydropyridi ne, 2-acetyl-2-thiazoline, and 5-acetyl-2,3-dihydro-4H-thiazine: extraordinary Maillard flavor compounds" CHEMICAL REVIEWS JUN 2006,, vol. 106, no. 6, 1 juin 2006 (2006-06-01), pages 2299-2319, XP002564375 cité dans la demande
- SINGH P N D ET AL: "An efficient one-pot synthesis of pyrrolines and tetrahydropyridines from their chloro-precursors via in situ aza-Wittig reaction" TETRAHEDRON LETTERS, ELSEVIER, AMSTERDAM, NL, vol. 46, no. 24, 13 juin 2005 (2005-06-13) , pages 4213-4217, XP025386173 ISSN: 0040-4039 [extrait le 2005-06-13]
- FAVINO T F ET AL: "Penicillin Acylase-Mediated Synthesis of 2-Acetyl-1-pyrroline and of 2-Propionyl-1-pyrroline, Key Roast-Smelling Odorants in Food. Inclusion Complexes with ss-Cyclodextrin and Their NMR and MS Characterization" JOURNAL OF ORGANIC CHEMISTRY, AMERICAN CHEMICAL SOCIETY, EASTON.; US, vol. 61, 1 janvier 1996 (1996-01-01), pages 8975-8979, XP002186606 ISSN: 0022-3263 cité dans la demande
- DE KIMPE N ET AL: "Syntheses of the Principal Bread Flavor Component, 6-Acetyl-1,2,3,4-tetrahydropyridine, and Acetal Protected Precursors" TETRAHEDRON, ELSEVIER SCIENCE PUBLISHERS, AMSTERDAM, NL, vol. 51, no. 8, 20 février 1995 (1995-02-20), pages 2387-2402, XP004104813 ISSN: 0040-4020
- DE KIMPE N ET AL:: "Novel Syntheses of the Major Flavor Components of Bread and Cooked Rice." JOURNAL OF AGRICULTURAL AND FOOD CHEMISTRY, vol. 44, no. 6, 18 juin 1996 (1996-06-18), pages 1515-1519, XP002566090 AMERICAN CHEMICAL SOCIETY. cité dans la demande
- PETER SCHIEBERLE ET AL.: "Quantitative Analysis of Aroma compounds in Wheat and Rye Bread Crusts Using a Stable Isotope Dilution Assay" JOURNAL OF AGRICULTURAL AND FOOD CHEMISTRY, vol. 35, no. 2, mars 1987 (1987-03), pages 252-257, XP002566091 AMERICAN CHEMICAL SOCIETY. cité dans la demande
- TYLER J. HARISSON ET AL.: "An Expeditious, High-Yielding Construction of the Food Aroma Compounds 6-Acetyl-1,2,3,4-tetrahydropyridine and 2-Acetyl-1-pyrroline" JOURNAL OF ORGANIC CHEMISTRY., vol. 2005, no. 70, 23 décembre 2005 (2005-12-23), pages 10872-10874, XP002566092 USAMERICAN CHEMICAL SOCIETY, WASHINGTON, DC. cité dans la demande

## Description

La présente invention concerne l'utilisation de dérivés cétals comme précurseurs stables dans une composition aromatisante. La présente description divulgue un procédé de synthèse de la 2-acétyl-1-pyrroline et analogues ainsi que l'utilisation d'analogues deutérés comme étalon interne dans un procédé de dosage.

La 2-acétyl-1-pyrroline (**1**) (nommée ci-après 2-AP) et son analogue à 6 chaînons, la 6-acétyl-1,2,3,4-tétrahydropyridine (**2**) (nommée ci-après 6-ATHP) en équilibre avec son tautomère la 6-acétyl-2,3,4,5-tétrahydropyridine (**3**), jouent un rôle majeur dans l'arôme des produits de cuisson d'aliments à base de céréales.

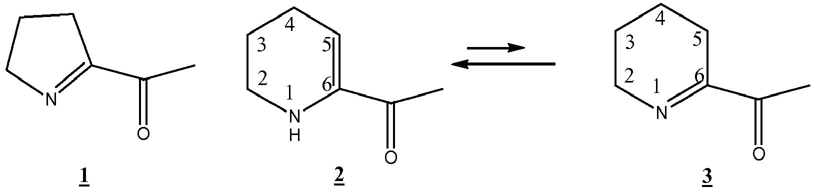

La 2-AP est une molécule volatile, présentant l'odeur de popcorn, de riz basmati et de pain grillé. Elle est un des composés caractéristiques des riz parfumés (Paule et Powers, J. Food Sci., 1989, 54, 343-346), variétés de riz issues de populations génétiques très restreintes et ne pouvant être cultivées hors de leur aire d'origine. Elle a été également détectée dans une large variété d'autres produits céréaliers mais également dans plusieurs produits alimentaires d'origine végétale et animale. Cette molécule est également formée par des réactions de Maillard (notamment à partir de la proline et de l'acide glutamique) pendant la cuisson du riz ou d'autres aliments à base de céréales tels que le pain grillé, le pop corn, les graines de sésame, etc. Ce composé, comme de nombreux hétérocycles, dispose d'une forte puissance olfactive, avec un seuil de détection de 0,02 ng/L dans l'air et de 0,1 µg/Kg dans l'eau (Adams et De Kimpe, Chem. Rev., 2006, 106, 2299-2319). La 2-AP peut donc être très utilisée dans l'aromatisation de produits alimentaires, comme les produits de boulangerie, les aliments grillés ou cuits, les boissons (thé, café), pour renforcer le caractère grillé ou encore comme modificateur dans les formulations aromatiques. Cependant, cette molécule est fragile et peut nécessiter d'être stabilisée dans certaines applications. Par ailleurs, la 2-AP est généralement présente à très faible concentration dans les aliments, elle a été évaluée de 6 à 90 ppb, dans 10 variétés différentes de riz cru, par un entraînement à la vapeur couplé à une extraction en continu (Buttery et coll., J. Agric. Food Chem., 1983, 31, 823-826). Cependant, l'instabilité de ce composé et sa faible teneur rend difficile son dosage, c'est la raison pour laquelle de nombreuses méthodes d'extractions et de dosage ont été expérimentées, variant suivant le milieu (Shieberle et Grosch, J. Agric. Food Chem., 1987, 35, 252-257 ; Yoshihashi et coll., J. Agric. Food Chem., 2002, 50(7), 2001-2004 ; Hoffman et Schieberle, J. Agric. Food Chem., 1998, 46, 616-619 et 2270-2277).

La 6-ATHP, quant à elle, est le composant aromatique majeur des produits de boulangerie. Cette molécule a également été identifiée dans les chips, les popcorns et la croûte de pain (Demyttenaere et coll., The chemistry of the most important Maillard flavor compounds of bread and cooked rice, In "Heteroatomic aroma compounds", ACS symposium series 826, Ed. Reineccius GA., Reineccius TA., Washington, 2002, 150-160). Elle présente également une odeur grillée typique, proche de celle du popcorn, avec néanmoins une puissance olfactive moindre que celle de son homologue inférieur : 0,06 ng/L dans l'air et 1 µg/Kg dans l'eau (Adams et De Kimpe, Chem. Rev., 2006, 106, 2299-2319).

Dans la même famille, on peut citer également la 2-propionyl-1-pyrroline (2PP) ou son homologue supérieur, la 2-propionyl-3,4,5,6-tétrahydropyridine, qui présentent une odeur caractéristique d'aliments grillés (Adams et De Kimpe, Chem. Rev., 2006, 106, 2299-2319).

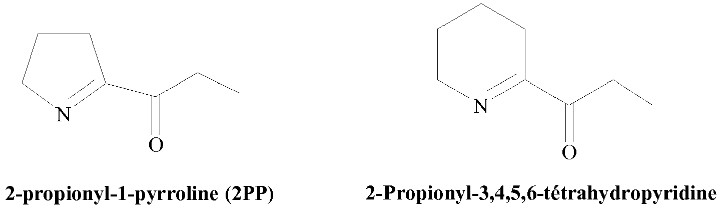

La première synthèse de la 2-AP a été réalisée par l'équipe de Buttery (Buttery et coll., J. Agric. Food Chem., 1983, 31, 823-826) par oxydation de la 2-(1-hydroxyéthyl)-pyrrolidine en présence d'un large excès de carbonate d'argent sur célite. Cependant, cette synthèse présente l'inconvénient d'utiliser un catalyseur assez coûteux, le rhodium/alumine. De plus, un marquage de la molécule par le deutérium est difficilement envisageable par une telle voie de synthèse.

Deux voies possibles de préparation de la 2-AP ont également été décrites par De Kimpe (De Kimpe et coll., J. Agric. Food Chem., 1993, 41, 1458-1461). La première voie utilise la pyrrolidine comme produit de départ et conduit à la formation de la 2-AP ou à son analogue deutéré (avec l'utilisation de CD₃MgI sur le dérivé nitrile). Cette synthèse présente cependant l'inconvénient d'utiliser un produit relativement dangereux qui est l'acide cyanhydrique. La deuxième voie de synthèse a été développée à partir de la proline mais la séparation des produits finaux à partir du mélange obtenu est difficile à optimiser. En 1991, De Kimpe (EP 0 436 481) décrivait déjà la synthèse la 2-AP à partir de la pyrrolidine mais aussi la préparation de la 6-ATHP à partir de la pipéridine selon un même processus d'oxydation de l'amine en imine suivi d'une cyanation, puis oxydation en 2-cyano-1-azacycloalcénes et pour finir réaction avec un réactif de Grignard.

La 2-AP a également été synthétisée par Rewicki et coll. (Progress in Flavor Precursor Studies; Schreier P., Winterhalter P. Eds.; Allured Publishing Corp.: Carol Stream, 1993, p 301) par une cyclisation intramoléculaire de type aza-Wittig sur l'azidodicétone. Ce précurseur dicarbonylé γ-azido est formé par une séquence de réactions impliquant l'addition de cyanure de triméthylsilyle sur un aldéhyde α,β-insaturé, l'α-déprotonation du nitrile suivie d'une alkylation, d'une désilylation et d'une ozonolyse. Cependant, cette synthèse est difficile à mettre en oeuvre et utilise des réactifs potentiellement toxiques. De même, la 2-acétyl-3,4,5,6-tetrahydropyridine a pu être préparée en **1993** par Rewicki et coll. (DE 42 17 395) lors d'une réaction de type aza-Wittig de cyclisation de la 7-azidoheptan-2,3-dione obtenue par une synthèse multiétapes. De Kimpe et Stevens (Tetrahedron, 1995, 51, 2387-2402) ont aussi développé cette approche consistant en une cyclisation de type aza-Wittig à partir d'un précurseur azoture formé à partir du dérivé chloré, à savoir la 7-chloro-2,2-diméthoxyheptan-3-one. Singh et coll. (Tetrahedron Letters, 2005, 46, 4213-4217) ont, sur le même principe, décrit une synthèse de la 6-ATHP à partir du même intermédiaire halogéné, la 7-chloro-2,2-diméthoxyheptan-3-one. Dans cette étude plus récente la réaction s'effectue par contre sous activation microondes.

De Kimpe et Keppens (J. Agric. Food Chem., 1996, 44 (6), 1515-1519) ont également développé un procédé de synthèse de la 2-AP à partir d'une diimine en position vicinale obtenue à partir de la butane-2,3-dione et de l'isopropylamine en présence de chlorure de titane. Elle permet d'obtenir la 6-amino-2,3-hexadione qui se cyclise spontanément pour conduire à la 2-AP accompagnée de la 6-méthyl-2,3,4,5-tétrahydropyridin-5-one. L'utilisation de cette méthode de synthèse est cependant limitée par la difficulté de séparation de ces deux composés.

La même année, Favino et coll. (J. Org. Chem., 1996, 61, 8975) ont développé une autre méthode de synthèse de la même α-dione aminée en position terminale, protégée par un groupement benzyloxy. L'hydrolyse enzymatique de ce composé par une acylase immobilisée conduit à l'amine primaire qui se cyclise spontanément en 2-AP. On peut cependant supposer que la cyclisation ne conduit pas exclusivement à la 2-AP comme dans la méthode décrite précédemment.

Hofinann et Schieberle (J. Agric. Food Chem., 1998, 46, 616-619 et 2270-2277) proposent une nouvelle méthode de synthèse à partir de la proline protégée par un groupement *t*-butyloxycarbonyle (Boc). Ils réalisent la synthèse, en trois étapes, de la 2-acétylpyrrolidine par formation d'un thioester, introduction du groupement méthyle par addition de bromure de méthyl magnésium, puis déprotection du groupement aminé par l'acide trifluoroacétique. La 2-acétylpyrrolidine, obtenue sous forme de sel de trifluoroacétate, s'oxyde spontanément en 2-acétyl-1-pyrroline après retour à pH 7 par addition d'une solution basique. Cette synthèse présente cependant l'inconvénient d'utiliser des magnésiens, composés qui sont facilement hydrolysables et donc délicats à manipuler.

Une autre voie d'obtention de la 2-acétyl-1-pyrroline faisant intervenir une étape de protection de l'azote du cycle a été proposée récemment par Harrison et Dake (J. Org. Chem., 2005, 70, 10872-10874). Ces auteurs proposent une stratégie de synthèse à partir de la pyrrolidin-2-one faisant intervenir une ouverture du cycle par un vinyl lithium puis cyclisation en milieu acide. La 2-AP est libérée après déprotection du groupement aminé, accompagnée de la 6-méthyl-2,3,4,5-tétrahydropyridin-5-one. L'utilisation de cette méthode de synthèse est cependant limitée par la difficulté de séparation de ces deux composés.

Fuganti et coll. (Tetrahedron, 2007, 63, 4762-4767) décrivent une méthode de synthèse des 2-AP et 6-ATHP par oxydation lors de l'étape clé finale de précurseurs imines cycliques. L'inconvénient de cette synthèse est l'utilisation d'un oxydant toxique le dioxyde de sélénium.

La demande de brevet EP 0 545 085 décrit une voie de synthèse à partir du N-(triméthylsilyl)-butyrolactame en présence de 1-éthoxyvinyl lithium à très basse température suivie d'une hydrolyse acide pendant 7 jours ce qui conduit à la formation de la 2-acétyl-1-pyrroline et de son tautomère difficilement séparables.

Le brevet US 6 723 856 revendique une voie de synthèse de la 2-acétyl-1-pyrroline en 3 étapes à partir de la proline. La synthèse passe par son ester méthylique qui est oxydé à 0°C pour former le noyau pyrroline, puis l'addition d'un magnésien conduit au composé désiré. Comme dans le cas de la méthode décrite par De Kimpe et coll. en 1993, on peut s'attendre à la formation de plusieurs produits d'addition lors de la dernière étape et l'utilisation de bromure de méthyl magnésium deutéré conduirait à une molécule marquée en position échangeable.

En 2007, Yukawa et Murakami (JP 2007/153785) proposent une autre voie de synthèse à partir de la proline faisant intervenir également une estérification méthylique en présence de SOCl₂ à -5-10°C, suivie d'une N-chloration par Me₃COCl, puis d'une déshydrochloration en présence de triéthylamine, et finalement d'une réaction de Grignard par MeMgI. Le brut de la réaction est repris dans l'éthanol et soumis à une distillation azéotropique. Il est à noter cependant que l'étape de N-chloration est délicate à mettre en oeuvre.

Ainsi, les méthodes de synthèse de la 2-AP de l'art antérieur présentent plusieurs inconvénients : tout d'abord certaines méthodes utilisent des produits toxiques qui sont difficilement envisageables pour une utilisation des produits finaux dans le domaine agroalimentaire, le produit final est souvent difficilement séparable d'autres produits formés lors de la dernière étape (tel que son tautomère) et enfin, certaines synthèses ne permettent pas de donner accès à des analogues marqués isotopiquement dans une position non échangeable.

Les inventeurs de la présente invention ont ainsi développé une nouvelle méthode de synthèse permettant de s'affranchir de ces inconvénients, et donc facile à mettre en oeuvre avec un faible coût, un minimum d'étapes et une purification aisées des molécules finales, n'employant pas de produits toxiques ou prohibés par l'industrie agroalimentaire et utilisant des composés naturels comme produit de départ (notamment l'acide glutamique ou l'acide 2-aminoadipique). Ce procédé de synthèse présente l'intérêt supplémentaire de donner facilement accès à des composés marqués isotopiquement (avec du deutérium) pour une utilisation notamment comme étalon interne dans un procédé de dosage. Il met en oeuvre par ailleurs des dérivés cétals, comme intermédiaires de synthèse, qui représentent des précurseurs stables des composés aromatisants permettant de résoudre le problème de stabilité rencontré avec ces molécules. En effet, les dérivés cétals peuvent être conservés de manière stable à l'abri de l'air et de l'humidité, puis peuvent libérer progressivement les molécules aromatisantes, par action de l'eau suivie de celle de l'oxygène.

La présente description divulgue un procédé de synthèse d'un composé de formule (I) suivante : 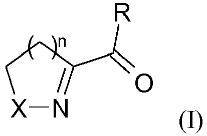 pour laquelle :
- R représente un groupe méthyle ou éthyle,
- n représente 1 ou 2, et
- X représente un groupe CH₂ ou CD₂,
à partir d'un composé de formule (II) suivante : 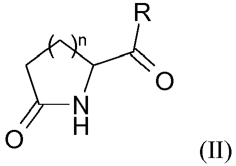 pour laquelle R et n sont tels que définis ci-dessus,
comprenant les étapes successives suivantes :
(a) éventuellement protection de la fonction carbonyle exocyclique du composé de formule (II) pour donner un composé de formule (III) suivante : 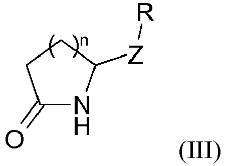 pour laquelle :
   - R et n sont tels que définis ci-dessus, et
   - Z représente un groupement carbonyle protégé, notamment sous la forme d'un cétal de formule C(AR¹)(AR²), où A représente un atome d'oxygène ou de soufre et R¹ et R² représentent, indépendamment l'un de l'autre, un groupe (C₁-C₆)alkyle, tel qu'un groupe éthyle, ou R¹ et R² forment ensemble une chaîne - (CH₂)ₚ reliant les atomes A qui les portent avec p représentant 2 ou 3,
(b) réduction du composé de formule (II) ou de formule (III) tels que définis ci-dessus en présence d'ions hydrures ou deutérures, et notamment en présence de LiAlH₄ ou LiAlD₄, pour donner un composé de formule (IV) suivante : 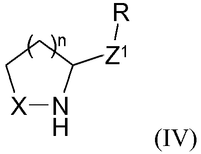 pour laquelle X, n et R sont tels que définis ci-dessus, et Z¹ représente un groupe Z tel que défini ci-dessus ou un groupe CHOH ou CDOH,
(c) éventuellement déprotection de la fonction carbonyle exocyclique protégée du composé de formule (IV) obtenu à l'étape précédente pour lequel Z¹ = Z, pour donner un composé de formule (V) suivante : 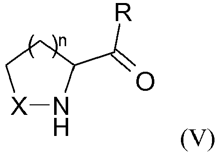 pour laquelle X, n et R sont tels que définis précédemment, et
(d) oxydation du composé de formule (IV) ou de formule (V) tels que définis ci-dessus, notamment en présence d'oxygène, pour donner un composé de formule (I).

Par « (C₁-C₆)alkyle », on entend, au sens de la présente invention, une chaîne hydrocarbonée saturée, linéaire ou ramifiée, comprenant 1 à 6 , de préférence 1 à 4, atomes de carbone. Les exemples de groupes alkyle comprennent, mais sans y être limité, les groupes méthyle, éthyle, n-propyle, isopropyle, sec-butyle, tert-butyle, n-butyle, isobutyle, n-pentyle et n-hexyle. Il s'agira en particulier d'un groupe éthyle ou méthyle.

### Etape (a) :

Cette étape permet de protéger la fonction carbonyle exocyclique pour éviter d'être réduite lors de l'étape (b) suivante.

Par « groupement protégé », on entend désigner, au sens de la présente invention, un groupement qui permet de bloquer sélectivement un site réactif dans un composé multifonctionnel de telle sorte qu'une réaction chimique peut être effectuée sélectivement au niveau d'un autre site réactif non protégé dans la signification classiquement associée à celui-ci en chimie de synthèse.

Par « groupement carbonyle protégé », on entend, au sens de la présente invention, tout groupe qui permet de protéger une fonction carbonyle, et plus particulièrement une fonction cétone dans le cas présent, contre les réactions indésirables tels que les groupes décrits dans Greene, « Protective Groups In Organic synthesis », (John Wiley & Sons, New York (1981)) et Harrison et al. « Compendium of Synthetic Organic Methods », Vols. 1 à 8 (J. Wiley & sons, 1971 à 1996). Il pourra s'agir en particulier d'un groupement cétal ou thiocétal de formule C(AR¹)(AR²) telle que définie ci-dessus. De préférence, A représentera un atome d'oxygène. Le groupement cétal pourra en particulier être C(OEt)₂.

### Etape (b) :

Les conditions de réalisation d'une telle étape de réduction sont bien connues de l'homme du métier. La réduction de la fonction amide, qui s'accompagnera de la réduction de la fonction cétone, si celle-ci n'est pas protégée, peut être réalisée en présence d'ions hydrures ou deutérures.

Les réactifs utiles pour effectuer cette réduction peuvent être en particulier LiAlH₄ ou LiAlD₄.

Avantageusement, cette réaction est réalisée dans un solvant comme l'éther diéthylique, de préférence à 0°C.

L'utilisation d'ions deutérures permet d'introduire sur la molécule deux atomes de deutérium en alpha de l'atome d'azote. La présence de tels atomes pourra être utile dans un procédé de dosage comme décrit ci-après, d'autant plus que ces atomes ne sont pas échangeables avec des atomes d'hydrogène (contrairement à la position en alpha du carbonyle) ce qui permet de maintenir le marquage isotopique.

### Etape (c) :

L'étape (c) ne sera réalisée que si l'étape (a) a été réalisée, c'est-à-dire si le carbonyle exocyclique a été protégé. Les conditions de réalisation de cette étape dépendront donc de la nature du groupe choisi pour protéger la cétone et sauront être déterminées par l'homme du métier.

Dans le cas où la cétone aurait été protégée sous forme de cétal de formule C(AR¹)(AR²) telle que définie ci-dessus, la déprotection pourra se faire par traitement en milieu acide, notamment en présence d'acide chlorhydrique aqueux.

### Etape (d) :

Les conditions d'oxydation de la fonction amine en imine et le cas échéant de l'alcool en cétone sont bien connues de l'homme du métier.

Ainsi, l'oxydation simultanée de l'amine et de l'alcool peut être réalisée par une réaction de Dess-Martin. Cette réaction se fait avantageusement sous atmosphère inerte, notamment sous une atmosphère d'argon ou d'azote, et avantageusement à température ambiante, notamment dans un solvant tel que l'éther diéthylique.

Dans le cas où la fonction cétone exocyclique est intacte, les inventeurs ont pu constater que le composé ainsi obtenu s'oxydait spontanément à l'air libre (c'est-à-dire par l'oxygène de l'air) ou par bullage d'oxygène dans un milieu réactionnel à pH = 7.

Le composé final ainsi obtenu pourra être extrait du milieu réactionnel par des techniques bien connues de l'homme du métier et être purifié si nécessaire.

Les produits obtenus étant des composés très volatils, ils pourront être isolés et purifiés par distillation.

Le composé de formule (II), utilisé comme produit de départ dans le procédé ci-dessus, peut lui-même être préparé à partir d'un composé de formule (VI) suivante : 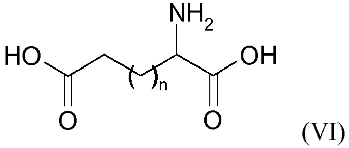 pour laquelle n est tel que défini ci-dessus,
par une réaction de Dakin-West en présence d'un anhydride de formule (RCO)₂O, avec R tel que défini ci-dessus, suivie d'un traitement basique.

Ainsi, la synthèse pourra être effectuée à partir de l'acide glutamique lorsque n = 1 ou à partir de l'acide 2-aminoadipique lorsque n = 2, qui sont des composés disponibles commercialement et d'origine naturelle.

Les conditions de réalisation de la réaction de Dakin-West sont bien connues de l'homme du métier. Cette réaction permet la cyclisation du composé de formule (VI) en cycle azoté à 5 (quand n = 1) ou 6 (quand n = 2) chaînons, avec acylation, et en particulier acétylation, simultanée en position 1 et 5 (quand n = 1) ou 6 (quand n = 2).

Cette réaction est réalisée avantageusement en milieu basique, notamment en présence de triéthylamine, dans l'anhydride acétique (pour R = Me) ou propanoïque (pour R = Et), en présence d'une quantité catalytique de DMAP (N,N-diméthyl-4-aminopyridine). De préférence, la réaction sera réalisée à chaud, à une température d'environ 60°C.

Cette réaction conduisant à l'obtention du dérivé N-acylé, il est alors nécessaire d'effectuer une étape d'hydrolyse pour obtenir la fonction amine libre, en particulier par un traitement basique, notamment en présence de K₂CO₃, comme indiqué sur le schéma réactionnel ci-dessous.

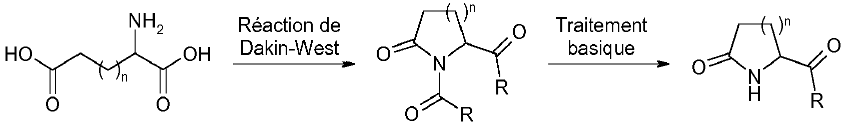

De manière avantageuse, le procédé comprendra les étapes successives suivantes :
(a1) protection sous forme d'un cétal de la fonction carbonyle exocyclique d'un composé de formule (II) tel que défini ci-dessus,
   pour donner un composé de formule (IIIa) suivante : 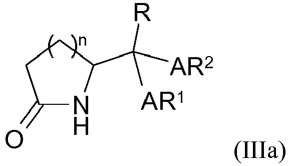 pour laquelle n, A, R, R¹ et R² sont tels que définis ci-dessus,
(b1) réduction du composé de formule (IIIa) obtenu à l'étape précédente en présence d'ions hydrures ou deutérures, et notamment en présence de LiAlH₄ ou LiAlD₄, pour donner un composé de formule (IVa) suivante : 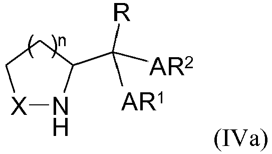 pour laquelle X, A, n, R, R¹ et R² sont tels que définis ci-dessus,
(c1) déprotection de la fonction carbonyle exocyclique protégée du composé de formule (IVa) obtenu à l'étape précédente pour donner un composé de formule (V) tel que défini ci-dessus, et
(d1) oxydation du composé de formule (V) obtenu précédemment, notamment en présence d'oxygène, pour donner un composé de formule (I).

Les remarques indiquées ci-dessus pour les étapes (a), (b), (c) et (d) s'appliquent respectivement aux étapes (a1), (b1), (c1) et (d1) décrites ci-dessus.

Ce procédé est particulièrement bien adapté pour la préparation des composés de formule (I) pour lesquels R représente un groupe méthyle et en particulier pour les composé suivants : la 2-acétyl-1-pyrroline, la 2-acétyl-1-pyrroline-5,5-d₂ et la 6-acétyl-1,2,3,4-tétrahydropyridine.

La présente description divulgue également un composé de formule (la) suivante : 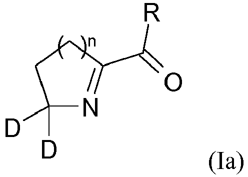 pour laquelle :
- R représente un groupe méthyle ou éthyle, et
- n représente 1 ou 2.

Un tel composé pourra être utile comme étalon interne dans une méthode de dosage comme décrit ci-dessous et pourra être en particulier la 2-acétyl-1-pyrroline-5,5-d₂.

La présente description divulgue également l'utilisation d'un composé de formule (la) tel que décrit ci-dessus, en tant qu'étalon interne dans un procédé de dosage d'un composé de formule (Ib) suivante : 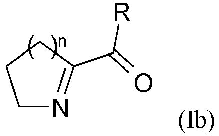 pour laquelle :
- R représente un groupe méthyle ou éthyle, et
- n représente 1 ou 2.

La particularité des analogues deutérés de formule (la) est leur deutération sur le cycle, qui évite les réactions d'échange, telles que celles observées avec des composés deutérés en α du carbonyle exocyclique, c'est-à-dire sur le radical R, et qui engendre la formation, en spectrométrie de masse, de fragments caractéristiques, faciles d'utilisation dans les méthodes de dosage par dilution isotopique. Les composés de formule (la) peuvent donc être utilisés comme étalons internes dans le dosage des hétérocycles naturels de formule (Ib) dans des milieux naturels complexes.

En particulier, la 2-acétyl-1-pyrroline-5,5-d₂ pourra être utilisé comme étalon interne pour le dosage de la 2-acétyl-1-pyrroline, notamment dans le riz.

Ainsi, la présente description divulgue un procédé de dosage d'un composé de formule (Ib) tel que défini ci-dessus utilisant un composé de formule (la) tel que défini ci-dessus, comme étalon interne.

Une telle méthode de dosage par étalonnage interne est utilisée en chromatographie pour effectuer une analyse quantitative. Elle peut être appliquée à différents types de détecteurs, une sensibilité accrue étant néanmoins obtenue avec des détecteurs à spectromètre de masse, en ciblant des fragments ioniques spécifiques avec une acquisition en mode SIM (Selected Ion Monitoring) (K. Ellendt et al., SOFIW J. 2001, 127, 29-34) ou appliquée en chromatographie-tandem MS/MS. La méthode de dosage par étalonnage interne est basée sur la comparaison individuelle du pic obtenu pour le composé à évaluer au pic obtenu pour la substance étalon convenablement choisie et introduite en proportion connue dans le mélange à analyser. La quantité du composé à évaluer est alors déterminée grâce à une courbe de « réponse » de ce composé à doser par rapport à la substance étalon qui aura été calculée indépendamment et préalablement au dosage, cette étape nécessitant l'utilisation des deux molécules de référence (composé à doser et substance étalon) (E. Cicchetti et al., Flavour Fragrance Journal, 2008, 23, 450-459). Par ailleurs, dans le cas du dosage d'un composé présent dans une matrice complexe nécessitant une extraction spécifique (comme ce peut être le cas pour les composés de formule (I) divulgués dans la présente description), une quantification précise préconise l'utilisation d'un analogue marqué comme étalon interne. Cette technique, qui est désignée par la terminologie « Stable isotope dilution assay » (SIDA), permet d'éliminer l'effet matrice au cours de l'extraction et tout changement éventuel des conditions expérimentales.

Le composé de formule (Ib) pourra être en particulier la 2-acétyl-1-pyrroline avec pour étalon interne la 2-acétyl-1-pyrroline-5,5-d₂ comme composé de formule (la).

La présente invention a pour objet un composé de formule (IVa) suivante : 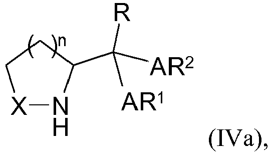 pour laquelle :
- R représente un groupe méthyle ou éthyle,
- n représente 1 ou 2,
- X représente un groupe CH₂ ou CD₂,
- A représente un atome d'oxygène ou de soufre, et
- R¹ et R² représentent, indépendamment l'un de l'autre, un groupe (C₁-C₆)alkyle, tel qu'un groupe éthyle, ou R¹ et R² forment ensemble une chaîne - (CH₂)ₚ reliant les atomes A qui les portent avec p représentant 2 ou 3.

De préférence, A représentera un atome d'oxygène et le groupe C(AR¹)(AR²) pourra être en particulier un groupe C(OEt)₂.

Le radical R pourra représenter en particulier un groupe méthyle et le composé de formule (IVa) pourra être choisi notamment parmi :

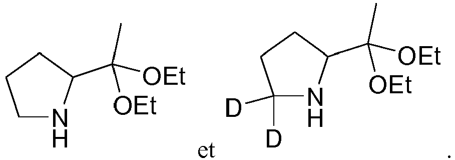

La présente invention a pour deuxième objet une composition aromatisante comprenant un composé de formule (IVa) tel que défini ci-dessus, pour lequel X = CH₂.

De préférence, A représentera un atome d'oxygène dans la formule (IVa) et le groupe C(AR¹)(AR²) pourra être en particulier un groupe C(OEt)₂.

Le radical R pourra représenter en particulier un groupe méthyle et le composé de formule (IVa) pourra être le composé suivant : 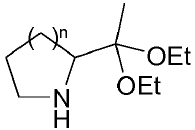 avec n et tel que défini ci-dessus et représentant notamment 1.

En effet, la faible stabilité de la 2-acétyl-1-pyrroline rend difficile son utilisation au niveau industriel (Zviely, Perfum. Flav., 2006, 31, 20-35). En revanche, son précurseur stable, l'aminocétal de formule (IVa), qui s'hydrolyse facilement en milieu faiblement acide ou neutre, avec oxydation spontanée en présence d'oxygène, peut être utilisé comme précurseur de molécules aromatisantes, notamment en agroalimentaire. Ainsi, tant que la composition aromatisante est préservée de tout risque d'hydrolyse acide et de tout contact avec l'air, et plus particulièrement avec l'oxygène de l'air, elle reste stable et peut facilement être stockée. Une fois mise en contact avec l'air, elle va pouvoir libérer progressivement et de manière contrôlée la 2-acétyl-1-pyrroline.

La présente invention a donc pour troisième objet l'utilisation d'un composé de formule (IVa) tel que défini ci-dessus, pour lequel X = CH₂ et A = O, pour aromatiser un produit alimentaire, le composé de formule (IVa) ayant avantageusement la formule suivante :

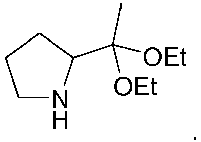

Comme indiqué ci-dessus, l'aromatisation est obtenue par formation d'un composé de formule (Ib) tel que défini ci-dessus, à partir du composé de formule (IVa) (par hydrolyse acide puis oxydation au contact de l'air), la formation du composé de formule (Ib) étant notamment contrôlée dans le temps.

La présente invention a également pour quatrième objet un procédé de préparation d'un composé de formule (IVa) tel que défini ci-dessus, caractérisé en ce qu'il comprend les étapes successives suivantes :
(a2) protection sous forme d'un cétal de la fonction carbonyle exocyclique d'un composé de formule (II) tel que défini ci-dessus,
   pour donner un composé de formule (IIIa) tel que défini ci-dessus, et
(b2) réduction du composé de formule (IIIa) obtenu à l'étape précédente en présence d'ions hydrures ou deutérures, et notamment en présence de LiAlH₄ ou LiAlD₄, pour donner un composé de formule (IVa) tel que défini ci-dessus.

Les remarques indiquées ci-dessus pour les étapes (a) et (b) s'appliquent respectivement aux étapes (a2) et (b2) décrites ci-dessus.

Par ailleurs, le composé de formule (II) utilisé comme produit de départ pour la synthèse des composés de formule (IVa) peut être préparé à partir d'un composé de formule (VI) tel que défini ci-dessus, par une réaction de Dakin-West en présence d'un anhydride de formule (RCO)₂O, avec R tel que défini ci-dessus, suivie d'un traitement basique.

Les remarques indiquées ci-dessus pour cette réaction de Dakin-West et ce traitement basique s'appliquent également au cas présent.

Ce procédé pourra être utilisé en particulier pour préparer un composé de formule (IVa) choisi parmi :

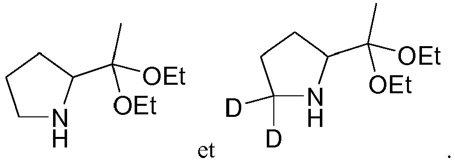

La présente invention pourra être mieux comprise à la lumière des exemples non limitatifs qui suivent.

### FIGURES

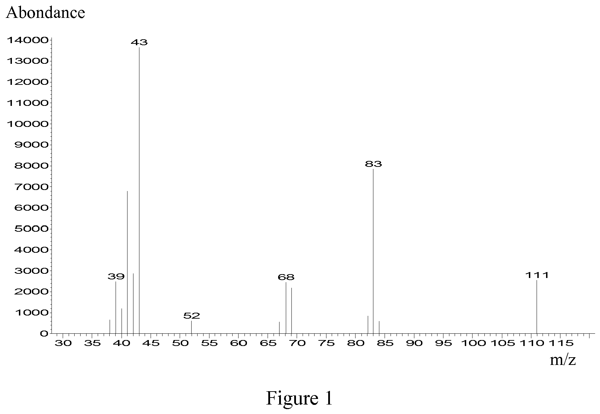

Les figures 1, 2, 3, 4 et 5 représentent les spectres de masse en impact électronique respectivement des composés la, 1b, 11, 12 et 13.

### EXEMPLES

### 1. Synthèse des composés de référence de formule (I)

### 1.1. Première méthode de synthèse de la 2-acétyl-1-pyrroline et de son analogue deutéré

Cette première synthèse a été effectuée selon le schéma réactionnel suivant :

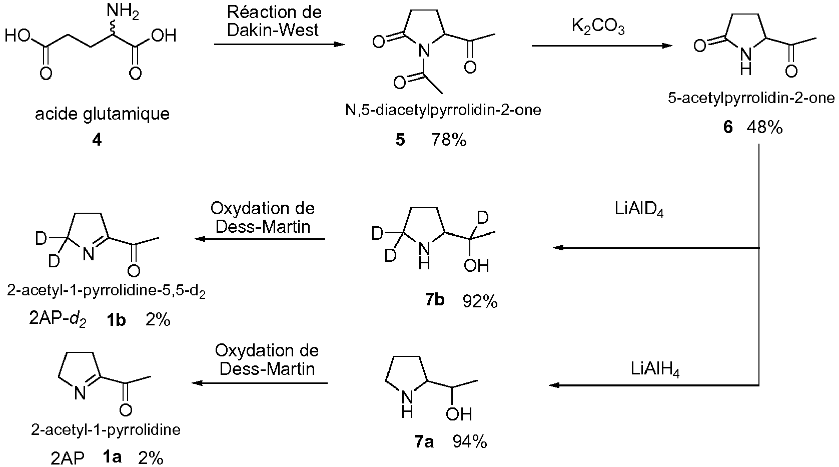

### • N, 5-diacétylpyrrolidin-2-one : 5

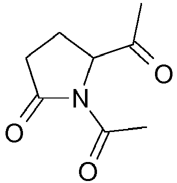

Dans un ballon de 500 mL sous argon, on met en présence d'acide L-glutamique **4** (21 g, 0.143mol), une quantité catalytique de N,N-diméthylaminopyridine (150 mg, 1.10⁻³mol), l'anhydride acétique (75 mL) et la triéthylamine préalablement distillée sur KOH (75 mL). Le milieu réactionnel est maintenu à 60°C pendant 16 heures. L'évolution de la réaction est suivie par CCM (éluant : acétate d'éthyle / éther de pétrole dans les proportions 8/2). Le brut de la réaction est concentré sous pression réduite (évaporateur rotatif et pompe à palettes) puis repris par 200 mL de dichlorométhane ; la phase organique est ensuite lavée à cinq reprises avec 100 mL d'eau désionisée puis traitée sur charbon animal. Le produit obtenu après évaporation du solvant est purifié par cristallisation dans un mélange éther de pétrole/CH₂Cl₂. Les cristaux sont rincés à l'éther de pétrole.

Le spectre RMN ¹H obtenu est conforme à la structure du composé indiqué.
**Rendement :** 78%
**SM: (ESI+) m/z**: 170.22 [M+H]⁺; 192.23 [M+Na]⁺; 361.23 [2M+Na]⁺

### • 5-acétylpyrrolidin-2-one : 6

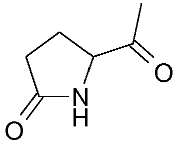

Dans un ballon, on traite le composé **5** (2,2 g, 13.10⁻³mol) par une solution de K₂CO₃ (5,6 g, 40.10⁻³Mol, dans 20 mL d'eau), sous agitation magnétique à température ambiante. L'avancement de la réaction est suivi par CCM (éluant : acétate d'éthyle/méthanol, 98/2). Après 3h30 d'agitation, la réaction est terminée. Le mélange réactionnel est neutralisé (pH=7) par ajout d'acide chlorhydrique 1N puis lyophilisé pendant 16h. Le résidu organique est ensuite extrait par CH₂Cl₂ (40 mL), filtré puis concentré sous pression réduite pour donner un liquide visqueux brun. La purification a été réalisée sur colonne de gel de silice (éluant : acétate d'éthyle/méthanol, 98/2). Le composé **6** ainsi purifié se présente sous forme d'une poudre blanche.

Les spectres RMN ¹H et ¹³C obtenus sont conformes à la structure du composé indiqué.
**Rendement** : 48%
**SM: (ESI+) m/z:** 128.20 [M+H]⁺; 150.09 [M+Na]⁺

### • 2-(1'-hydroxyéthyl)-pyrrolidine : 7a

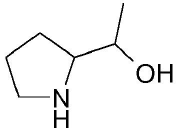

Dans un ballon sous argon, le composé **6** (209 mg, 1,65.10⁻³mol) est traité par 4 équivalents de LiAlH₄ dans l'éther éthylique (60 mL) préalablement distillé sur sodium. L'introduction de l'hydrure sur le composé **6** se fait dans un bain de glace. Le milieu réactionnel est ensuite porté au reflux de l'éther. L'avancement de la réaction est suivi par CCM (éluant : méthanol/acétate d'éthyle, 5/5). La réaction est terminée au bout de 3h. L'hydrure résiduel est décomposé par une solution aqueuse diluée d'HCl (addition de 4 mL d'une solution à pH=3). Après décantation, la phase aqueuse est extraite à l'éther éthylique (2 fois 20 mL). Les phases organiques sont ensuite réunies, séchées sur MgSO₄ anhydre et concentrées sous pression réduite. On obtient un liquide très visqueux incolore.

Le spectre RMN ¹H obtenu est conforme à la structure du composé indiqué.
**Rendement** : 92%.
**SM: (ESI+) m/z:** 115.85 [M+H]⁺, 138.15 [M+Na]⁺

### • 2-(1'-hydroxyéthyl)-pyrrolidine (deutéré) : 7b

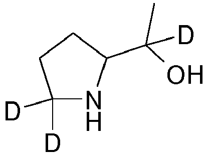

Dans un ballon sous argon, le composé **6** (209 mg, 1,65 10⁻³mol ; 1 équivalent) est traité par LiAlD₄ (276 mg, 6,58 10⁻³mol, 4 équivalents) dans l'éther éthylique (50 ml) préalablement distillé sur sodium. L'ajout de deutérure sur le composé **6** se fait dans un bain de glace. Le mélange réactionnel est ensuite porté au reflux de l'éther. L'avancement de la réaction est suivi par CCM (éluant : méthanol/acétate d'éthyle : 5/5). Après 3h de réaction, le LiAlD₄ résiduel est hydrolysé par un minimum d'eau acidulée. Après décantation, la phase aqueuse est extraite à l'éther éthylique (2 fois 20 ml). Les phases organiques sont réunies, séchées sur MgSO₄ anhydre puis concentrées sous pression réduite. On obtient un liquide visqueux incolore.

Le spectre RMN ¹H obtenu est conforme à la structure du composé indiqué.
**Rendement :** 94%.
**SM (ESI+)** : **m/z :** 118,85 [M+H]⁺, 141,15 [M+Na]⁺

### • 2-acétyl-1-pyrroline : 1a

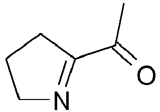

Dans 5 mL de CH₂Cl₂ introduits dans un ballon sous argon sont ajoutés 5 mL de réactif de Dess-Martin (1,51.10⁻³mol). Le composé **7a** (158mg, 1,37.10⁻³mol) dissout dans 2 mL de CH₂Cl₂ est incorporé goutte à goutte dans le milieu réactionnel. On contrôle l'avancement réactionnel par CCM (éluant : acétate d'éthyle/méthanol, 8/2). A la fin de la réaction (après 25 minutes), le milieu réactionnel est dilué par de l'éther éthylique (10 mL) préalablement distillé sur sodium.

Parallèlement, dans un second ballon, sont introduits 10 mL d'une solution de NaHCO₃ saturée contenant du bisulfite de sodium (2,5 g). Le mélange réactionnel est incorporé dans cette solution, placé sous agitation magnétique à température ambiante pendant 10 minutes puis dilué par de l'éther éthylique (10 mL). La phase organique est traitée par une solution de NaHCO₃ saturée (2 fois 10 mL) puis par de l'eau distillée (2 fois 10mL). Après décantation, la phase éthérée est séchée sur MgSO₄ anhydre. La concentration se fait à pression atmosphérique avec piégeage à froid (azote liquide) du composé **1a** qui est très volatil et qui est conservé dans l'éther éthylique à -20°C.

Les spectres de masse et RMN obtenus sont conformes à la structure du composé **1a.** Le spectre de masse en impact électronique est représenté sur la Figure 1.

### • 2-acétyl-1-pyrroline-5,5-d₂ : 1b

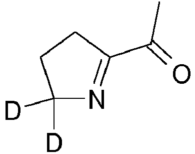

Le composé **1b** est préparé selon le même protocole que celui décrit pour la synthèse du composé **1a** excepté que l'on remplace le composé **7a** par le composé **7b**.

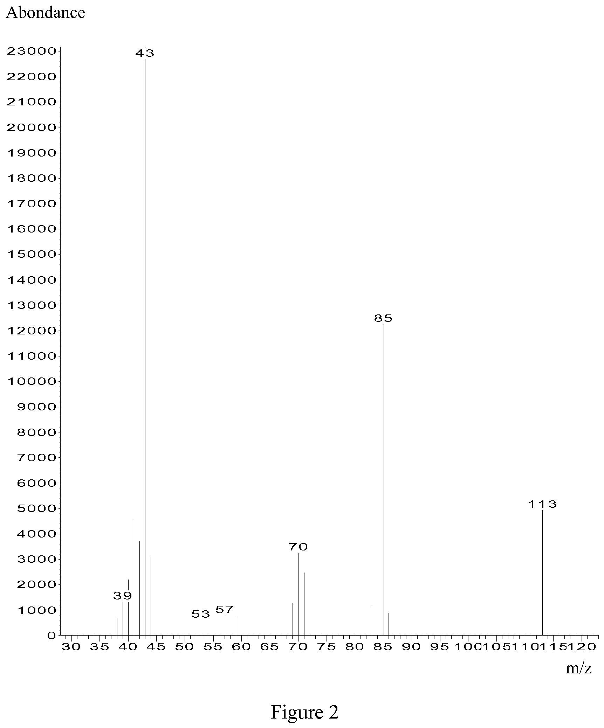

Les spectres de masse et RMN obtenus sont conformes à la structure du composé **1b.** Le spectre de masse en impact électronique est représenté sur la Figure 2.

### 1.2. Seconde méthode de synthèse de la 2-acétyl-1-pyrroline

Cette seconde méthode de synthèse a été réalisée selon le schéma réactionnel suivant :

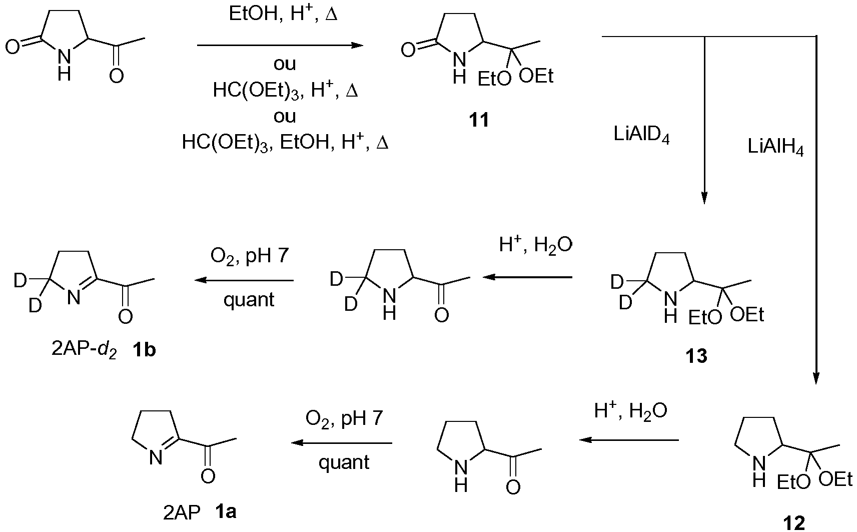

### • 5-(1',1'-diéthoxyéthyl)-pyrrolidin-2-one : 11

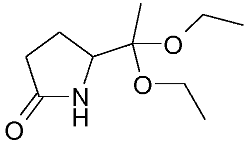

Dans un bicol et sous argon, sont introduits 127 mg (1 10⁻³mol =1 éq) de 5-acétylpyrrolidin-2-one **6** dans 1,1 ml de triéthylorthoformiate préalablement distillé sur KOH ; 330 mg de CaSO₄ et 146 mg de résine échangeuse de proton (Amberlyst 15) sont ajoutés dans le milieu. L'ensemble est placé sous agitation magnétique à température ambiante pendant 36 heures ; après addition de 30 mL d'éther éthylique, le milieu réactionnel est lavé rapidement avec 20 mL d'eau distillée. La phase organique est ensuite récupérée puis séchée sur MgSO₄. Après concentration sous pression réduite, le brut est chromatographié sur colonne de gel de silice (éluant : éther éthylique / éthanol, 96/4).

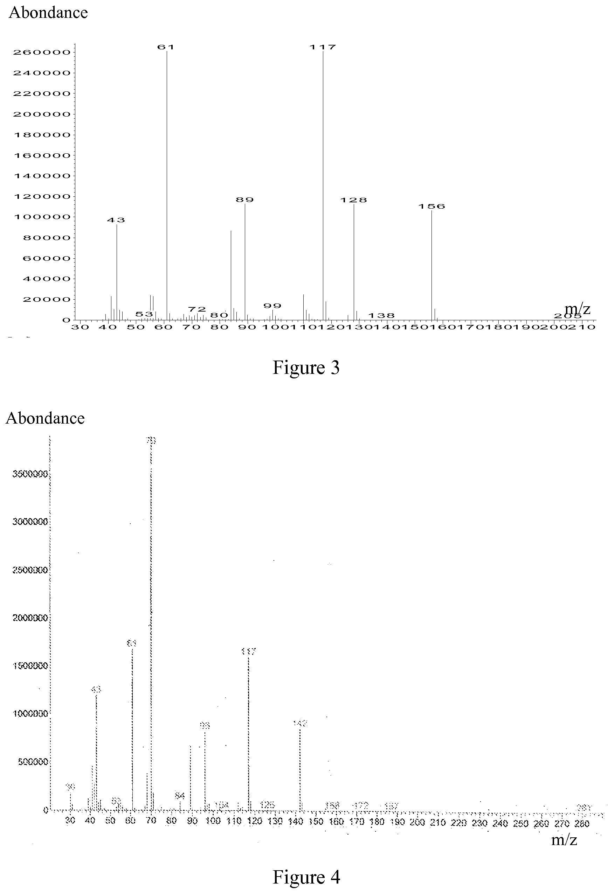

Les spectres RMN ¹H et ¹³C obtenus sont conformes à la structure du composé indiqué. Le spectre de masse en impact électronique est représenté sur la Figure 3.
**Rendement** : 36%.
**Rf** : 0,38 (éther éthylique / éthanol : 96/4).
**SM: (ESI+) m/z:** 224,28 [M+Na]⁺; 425,48 [2M+Na]⁺

### • 2-(1',1'-diéthoxyéthyl)-pyrrolidine : 12 (composé selon la présente invention)

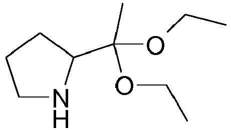

Dans un bicol de 100 mL et sous argon sont introduits 30 mL d'éther éthylique fraîchement distillé, puis 31 mg (0,81 10⁻³mol, 3 éq.) de LiAlH₄ en poudre. Puis, à l'aide d'une seringue graduée, sont ajoutés 56 mg (0,27 10⁻³mol, 1 éq.) du composé **11** préalablement dissout dans 2 mL d'éther éthylique. Le mélange réactionnel est laissé sous agitation à reflux pendant 12 heures ; l'hydrolyse du LiAlH₄ résiduel est ensuite réalisée avec 2 mL d'eau distillée à pH neutre. 20 mL d'éther éthylique sont ensuite ajoutés dans le milieu réactionnel qui est laissé sous agitation magnétique pendant 10 minutes. Après décantation, la phase aqueuse est extraite avec 3x5 mL d'éther éthylique. Les phases éthérées sont rassemblées et concentrées sous pression réduite. On obtient une huile jaunâtre.

Le spectre RMN ¹H obtenu est conforme à la structure du composé indiqué. Le spectre de masse en impact électronique est représenté sur la Figure 4.
**Rendement** : 82%.
**SM: (IE) m/z:** 70 (100), 61 (43), 117 (41), 13 (32),142 (22), 96 (21), 187 (M⁺, 2).

### • 2-(1',1'-diéthoxyéthyl)-pyrrolidine-5,5-d₂ : 13

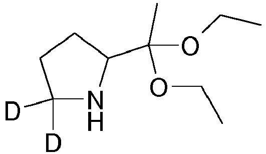

Ce composé a été préparé selon le protocole de synthèse du composé de la présente invention **12**, en remplaçant LiAlH₄ par LiAlD₄.

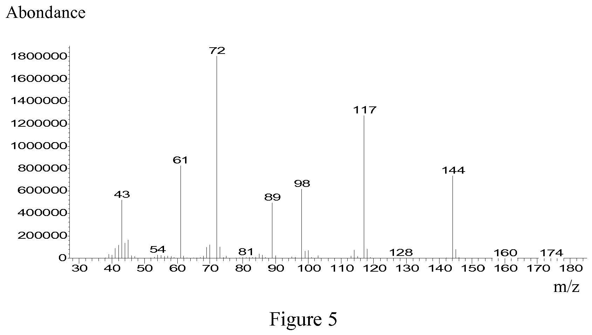

Le spectre de masse obtenu en impact électronique est représenté sur la Figure 5.

### • 2-acétyl-1-pyrroline : 1a

Dans un ballon de 50 mL sont introduits 38mg du composé de la présente invention **12** solubilisé dans 1 mL d'éther éthylique; après addition de 5 mL d'eau distillée, le mélange réactionnel est laissé sous agitation magnétique pendant 30 mn à température ambiante. Le pH de la solution est ensuite ramené à 3 par addition d'une solution aqueuse d'HCl 1N puis laissé sous agitation magnétique à température ambiante pendant 12 heures.

Le milieu réactionnel est ensuite ramené à pH 7 par addition d'une solution aqueuse de NaOH 1M; le ballon est placé dans un bain de glace et on fait barboter de l'oxygène dans le milieu réactionnel pendant 6 heures, sous agitation vigoureuse. On procède alors à une extraction avec 2 mL d'éther éthylique.

La phase organique, après séchage sur MgSO₄ est conservée à -20°C.

Les analyses GC et GC/MS confirment l'obtention de la 2-acétyl-1-pyrroline.
**Rendement** : 22%

### • 2-acétyl-1-pyrroline-5,5-d₂ : 1b

Le composé **1b** a également été obtenu selon le protocole décrit pour **1a** préparation du composé la en remplaçant le composé **12** par le composé **13.**

### 1.3. Synthèse de la 6-acétyl-1,2,3,4-tétrahydropyridine

Cette synthèse a été réalisée en utilisant le même protocole de synthèse qu'au point 1.1.

### • N,6-diacétyl-pipéridin-2-one : 8

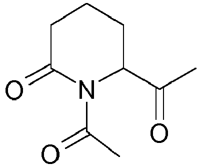

Dans un ballon de 50 mL, on introduit l'acide 2-aminoadipique racémique (2.3 g, 1,43.10⁻³ mol) ainsi que la triéthylamine (7,5 mL), l'anhydride acétique (7,5 mL) et une quantité catalytique de DMAP (15 mg). Le mélange est chauffé à 60°C pendant 24h ; l'avancement de la réaction est suivi par CCM (éluant : acétate d'éthyle/éther de pétrole, 8/2). Une fois la réaction terminée, le brut de réaction est concentré au maximum (évaporateur rotatif et pompe à palettes) puis lavé à l'eau (5 fois 20 mL). Les fractions aqueuses de rinçage sont extraites par CH₂Cl₂ (2 fois 20 mL). Les phases organiques sont réunies et concentrées. La purification de **8** est réalisée sur colonne de gel de silice (éluant : acétate d'éthyle/éther de pétrole, 8/2). Les fractions sont séchées, concentrées et le produit cristallise sous forme de paillettes jaune pâle.

Les spectres RMN ¹H et ¹³C obtenus sont conformes à la structure du composé indiqué.
**Rendement** : 25 %
**SM: (ESI+) m/z:** 184,25 [M+H]⁺, 206,12 [M+Na]⁺

### • 6-acétylpipéridin-2-one : 9

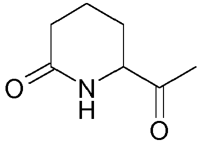

Le composé **8** est mis en présence de Na₂CO₃ (4 équivalents) dans de l'eau (20 mL). L'ensemble est placé sous agitation à température ambiante et l'avancement de la réaction est contrôlé par CCM (éluant : acétate d'éthyle/méthanol, 9/1). La réaction est totale au bout de 4h. Après neutralisation par HCl 1N (jusqu'à pH=7), l'ensemble est lyophilisé pendant 16h.

Le lyophilisat est repris dans du CH₂Cl₂ ; la solution est séchée sur MgSO₄ anhydre puis concentrée sous pression réduite.

Les spectres RMN ¹H et ¹³C obtenus sont conformes à la structure du composé indiqué.
**Rendement** : 42 %
**SM: (ESI+) m/z:** 142,03 [M+H]⁺, 164,13 [M+Na]⁺.

### • 2-(1'-hydroxyéthyl)-pipéridine : 10

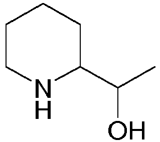

Le composé **9** obtenu précédemment est traité par LiAlH₄ dans l'éther éthylique anhydre (20 mL) sous atmosphère d'argon. L'hydrure d'aluminium et de lithium (4,5 équivalents) est ajouté au composé **9** (11,5mg, 8,16.10⁻⁴ mol) dans un ballon placé dans un bain de glace. Une fois l'addition terminée, le milieu réactionnel est porté au reflux de l'éther. L'avancement de la réaction est contrôlé par CCM (éluant : acétate d'éthyle/méthanol, 5/5). Au bout de 16 h la réaction est complète. L'hydrure en excès est détruit par une solution aqueuse acide (4 mL d'une solution aqueuse acidulée à pH=3). Après décantation de la phase éthérée, la phase aqueuse est à nouveau extraite par Et₂O (2 fois 20 mL). Les phases éthérées sont rassemblées, séchées sur MgSO₄ anhydre et concentrées sous pression réduite à l'évaporateur rotatif.

Le spectre RMN ¹H obtenu est conforme à la structure du composé indiqué.
**Rendement** : 52 %

### • 6-acétyl-1,2,3,4-tétrahydropyridine : 2 et 6-acétyl-2,3,4,5-tétrahydro pyridine : 3

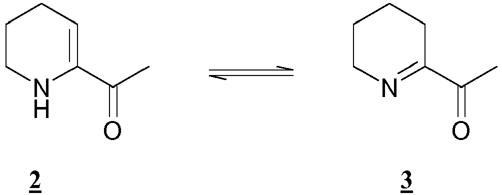

La réaction est réalisée en milieu anhydre sous atmosphère d'argon. Dans un bicol, on introduit du CH₂Cl₂ (3 mL) auquel on ajoute le réactif de Dess-Martin (4,50.10⁻⁴ mol) sous forme d'une solution à 0.3 M dans CH₂Cl₂ (soit 1,5 mL). Le composé **10** (53 mg, 4,1.10⁻⁴mol) précédemment préparé est mis en solution dans 1 mL de CH₂Cl₂ et additionné goutte au réactif de Dess-Martin. L'avancement de la réaction est contrôlé par CCM (éluant : acétate d'éthyle/méthanol, 8/2). Au bout de 20 minutes, la réaction n'évoluant plus, on rajoute 1 mL de réactif de Dess-Martin, ce qui permet d'obtenir, au bout de 30 minutes, la disparition complète de l'amino alcool.

Le mélange réactionnel est alors dilué avec 3 mL d'éther éthylique.

Une solution saturée de NaHCO₃ (3mL) est préparée et additionnée de 0,8g de bisulfite de sodium. Le mélange réactionnel est incorporé à cette solution et l'ensemble est placé sous agitation magnétique pendant 10 minutes, puis dilué par 3 mL d'éther éthylique.

La phase organique est décantée puis traitée à nouveau avec une solution de NaHCO₃ saturée (2 fois 10 mL), puis à l'eau (2 fois 10mL) ; après une nouvelle décantation, la phase éthérée est séchée sur MgSO₄ anhydre puis concentrée par microdistillation, à pression atmosphérique, avec piégeage à froid (N₂ liquide) des composés **2** et **3.**

### 2. Utilisation de la 2-AP-d₂ pour un dosage par étalonnage interne

La 2-acétyl-1-pyrroline-5,5-*d₂* (2AP- *d₂*) peut être utilisée pour la quantification de la 2-AP naturelle dans différents milieux selon une méthode de dosage par dilution isotopique stable (SIDA) ; la molécule étant volatile, la technique analytique utilisée sera préférentiellement la chromatographie en phase gazeuse qui pourra être couplée à un spectromètre de masse selon différents modes de fonctionnement tels que :
- ionisation chimique positive-spectrométrie de masse en tandem (ICP-SM/SM) : L'intérêt de la méthode est l'obtention d'un pic pseudomoléculaire intense [M + H]⁺, à 112 et 114 pour la 2AP et la 2AP*-d₂* qui se fragmentent pour donner respectivement les ions 70 et 72 (ions fils) correspondant à la partie cyclique de la molécule; la substitution par deux deutériums sur le cycle en position 5 permet d'observer une différence de deux unités sur chacun de ces ions qui peuvent être utilisés pour le tracé des courbes de calibration.
- impact électronique-mode SIM (selective ion monitoring) :
   Ce mode d'analyse permet de sélectionner les ions-cibles 83 et 85, caractéristiques respectivement de la 2AP et la 2AP-*d₂*, pour le tracé des courbes de calibration.

Ces méthodes de dosage ont l'avantage d'accéder à une excellente sensibilité (à titre d'exemple de l'ordre de 41 ng/kg de riz par la première méthode).

### ABREVIATIONS

- CCM: Chromatographie sur couche mince
- DMAP: N,N-Diméthyl-4-aminopyridine
- ESI: Ionisation par électrospray
- GC: Chromatographie en phase gazeuse
- GC/MS: Chromatographie en phase gazeuse couplée à un spectromètre de masse
- IE: Impact Electronique
- Rf: Rapport frontal
- RMN: Résonance magnétique nucléaire
- SIM: Selective ion monitoring
- SM: Spectre de masse

## Revendications

1. Composé de formule (IVa) suivante : 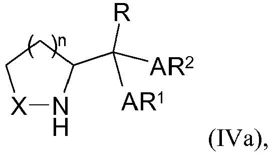 pour laquelle :
- R représente un groupe méthyle ou éthyle,
- n représente 1 ou 2,
- X représente un groupe CH₂ ou CD₂,
- A représente un atome d'oxygène ou de soufre, et
- R¹ et R² représentent, indépendamment l'un de l'autre, un groupe (C₁-C₆)alkyle ou R¹ et R² forment ensemble une chaîne - (CH₂)ₚ- reliant les atomes A qui les portent avec p représentant 2 ou 3.

2. Composé selon la revendication 1, **caractérisé en ce que** R¹ et R² représentent, indépendamment l'un de l'autre, un groupe éthyle ou R¹ et R² forment ensemble une chaîne - (CH₂)ₚ- reliant les atomes A qui les portent avec p représentant 2 ou 3.

3. Composé selon l'une quelconque des revendications 1 et 2, **caractérisé en ce que** A représente un atome d'oxygène.

4. Composé selon la revendication 3, **caractérisé en ce que** le groupe C(AR¹)(AR²) représente un groupe C(OEt)₂.

5. Composé selon l'une quelconque des revendications 1 à 4, **caractérisé en ce qu'**il est choisi parmi :

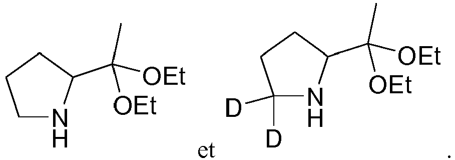

6. Composition aromatisante comprenant un composé de formule (IVa) selon l'une quelconque des revendications 1 à 5, pour lequel X = CH₂.

7. Composition aromatisante selon la revendication 6, **caractérisée en ce que** le composé de formule (IVa) est le composé de formule :

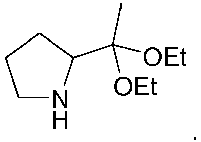

8. Utilisation d'un composé de formule (IVa) selon l'une quelconque des revendications 1 à 5, pour lequel X = CH₂, pour aromatiser un produit alimentaire.

9. Utilisation selon la revendication 8, **caractérisée en ce que** le composé de formule (IVa) est le composé de formule :

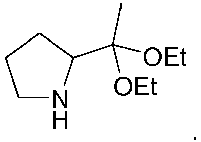

10. Utilisation selon l'une quelconque des revendications 8 et 9, **caractérisée en ce que** l'aromatisation est obtenue par formation d'un composé de formule (Ib) suivante : 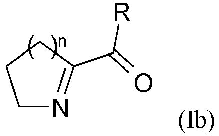 pour laquelle R et n sont tels que définis à la revendication 1,
à partir du composé de formule (IVa).

11. Utilisation selon la revendication 10, **caractérisée en ce que** la formation du composé de formule (Ib) est contrôlée dans le temps.

12. Procédé de préparation d'un composé de formule (IVa) tel que défini à la revendication 1, **caractérisé en ce qu'**il comprend les étapes successives suivantes :
(a2) protection sous forme d'un cétal de la fonction carbonyle exocyclique d'un composé de formule (II) suivante : 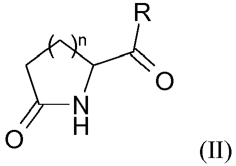 pour laquelle R et n sont tels que définis à la revendication 1,
pour donner un composé de formule (IIIa) suivante : 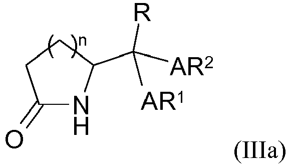 pour laquelle n, A, R, R¹ et R² sont tels que définis à la revendication 1,
(b2) réduction du composé de formule (IIIa) obtenu à l'étape précédente en présence d'ions hydrures ou deutérures pour donner un composé de formule (IVa) tel que défini à la revendication 1.

13. Procédé selon la revendication 12, **caractérisé en ce que** la réduction de l'étape (b2) est effectuée en présence de LiAlH₄ ou LiAlD₄.

14. Procédé selon l'une quelconque des revendications 12 et 13, **caractérisé en ce que** le composé de formule (II) est préparé à partir d'un composé de formule (VI) suivante : 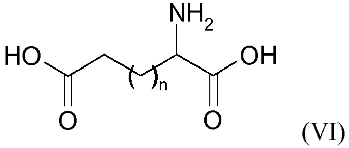 pour laquelle n est tel que défini à la revendication 1,
par une réaction de Dakin-West en présence d'un anhydride de formule (RCO)₂O, avec R tel que défini à la revendication 1, suivie d'un traitement basique.

15. Procédé selon l'une quelconque des revendications 12 à 14, **caractérisé en ce que** le composé de formule (IVa) est choisi parmi :

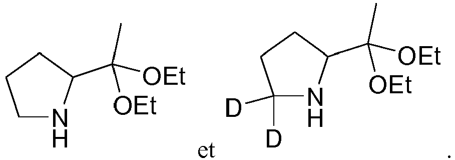

## Patentansprüche

1. Verbindung der folgenden Formel (IVa): 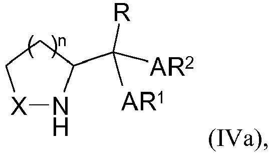 wobei:
- R eine Methyl- oder Ethylgruppe darstellt,
- n 1 oder 2 darstellt,
- X eine Gruppe CH₂ oder CD₂ darstellt,
- A ein Sauerstoff- oder Schwefelatom darstellt, und
- R¹ und R² unabhängig voneinander eine (C₁-C₆)Alkylgruppe darstellen oder R¹ und R² gemeinsam eine Kette (CH₂)ₚ bilden, die die Atome A verbindet, die sie tragen, wobei p 2 oder 3 darstellt.

2. Verbindung nach Anspruch 1, **dadurch gekennzeichnet, dass** R¹ und R² unabhängig voneinander eine Ethylgruppe darstellen oder R¹ und R² gemeinsam eine Kette (CH₂)ₚ bilden, die die Atome A verbindet, die sie tragen, wobei p 2 oder 3 darstellt.

3. Verbindung nach einem der Ansprüche 1 und 2, **dadurch gekennzeichnet, dass** A ein Sauerstoffatom darstellt.

4. Verbindung nach Anspruch 3, **dadurch gekennzeichnet, dass** die Gruppe C(AR¹) (AR²) eine Gruppe C(OEt)₂ darstellt.

5. Verbindung nach einem der Ansprüche 1 bis 4, **dadurch gekennzeichnet, dass** sie ausgewählt ist aus:

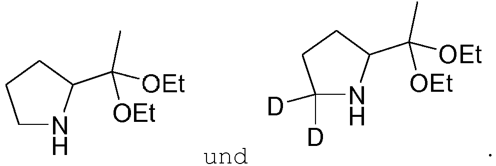

6. Aromatisierende Zusammensetzung, umfassend eine Verbindung der Formel (IVa) nach einem der Ansprüche 1 bis 5, wobei X = CH₂.

7. Aromatisierende Zusammensetzung nach Anspruch 6, **dadurch gekennzeichnet, dass** die Verbindung der Formel (IVa) die Verbindung der Formel ist:

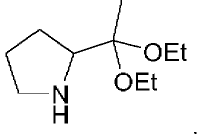

8. Verwendung einer Verbindung der Formel (IVa) nach einem der Ansprüche 1 bis 5, wobei X = CH₂, um ein Lebensmittelprodukt zu aromatisieren.

9. Verwendung nach Anspruch 8, **dadurch gekennzeichnet, dass** die Verbindung der Formel (IVa) die Verbindung der Formel ist:

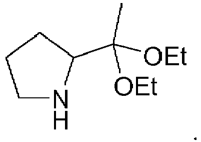

10. Verwendung nach einem der Ansprüche 8 und 9, **dadurch gekennzeichnet, dass** die Aromatisierung durch Bildung einer Verbindung der folgenden Formel (Ib) erhalten wird: 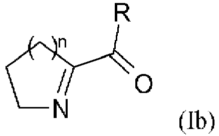
wobei R und n nach Anspruch 1 sind,
aus der Verbindung der Formel (IVa).

11. Verwendung nach Anspruch 10, **dadurch gekennzeichnet, dass** die Bildung der Verbindung der Formel (Ib) zeitlich überwacht wird.

12. Verfahren zur Herstellung einer Verbindung der Formel (IVa) nach Anspruch 1, **dadurch gekennzeichnet, dass** es die folgenden aufeinanderfolgenden Schritte umfasst:
(a2) Schützen, in Form eines Ketals, der exocyclischen Carbonylfunktion einer Verbindung der folgenden Formel (II): 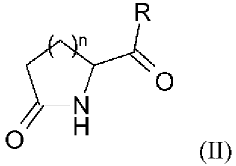 wobei R und n nach Anspruch 1 sind,
um eine Verbindung der folgenden Formel (IIIa) zu erhalten: 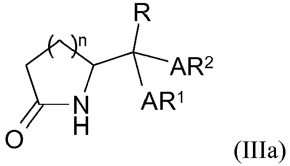 wobei n, A, R, R¹ und R² nach Anspruch 1 sind,
(b2) Reduzieren der Verbindung der Formel (IIIa) aus vorangehendem Schritt in Anwesenheit von Hydrid- oder Deuteridionen, um eine Verbindung der Formel (IVa) nach Anspruch 1 zu erhalten.

13. Verfahren nach Anspruch 12, **dadurch gekennzeichnet, dass** die Reduzierung von Schritt (b2) in Anwesenheit von LiAlH₄ oder LiAlD₄ durchgeführt wird.

14. Verfahren nach einem der Ansprüche 12 und 13, **dadurch gekennzeichnet, dass** die Verbindung der Formel (II) aus einer Verbindung der folgenden Formel (VI) hergestellt ist: 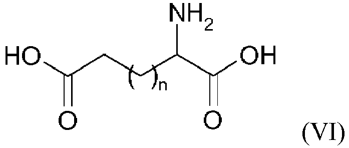
wobei n nach Anspruch 1 ist,
mit einer Dalin-West-Reaktion in Anwesenheit eines Anhydrids der Formel (RCO) ₂O mit R nach Anspruch 1, gefolgt von einer basischen Behandlung.

15. Verfahren nach einem der Ansprüche 12 bis 14, **dadurch gekennzeichnet, dass** die Verbindung der Formel (IVa) ausgewählt ist aus:

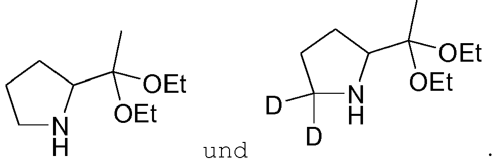

## Claims

1. A compound of the following formula (IVa): 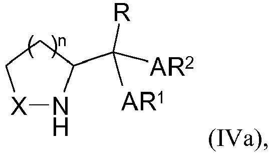 wherein:
- R is a methyl or ethyl group,
- n is 1 or 2,
- X is a CH₂ or CD₂ group,
- A represents an oxygen or sulfur atom, and
- R¹ and R² represent, independently from each other, a (C₁-C₆) alkyl group, or R¹ and R² taken together form a -(CH₂)ₚ- chain connecting the atoms A carrying them, where p is 2 or 3.

2. The compound according to claim 1, **characterized in that** R¹ and R² represent, independently from each other, an ethyl group, or R¹ and R² taken together form a - (CH₂)ₚ- chain connecting the atoms A carrying them, where p is 2 or 3.

3. The compound according to any one of claims 1 and 2, **characterized in that** A represents an oxygen atom.

4. The compound according to claim 3, **characterized in that** the group C(AR¹) (AR²) is a C(OEt)₂ group.

5. The compound according to any one of claims 1 to 4, **characterized in that** it is selected from:

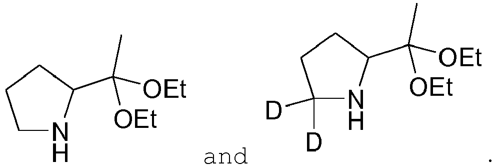

6. A flavoring composition comprising a compound of the formula (IVa) according to any one of claims 1 to 5, in which X=CH₂.

7. The flavoring composition according to claim 6, **characterized in that** the compound of formula (IVa) is the compound of the formula:

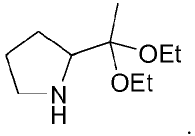

8. The use of a compound of the formula (IVa) according to any one of claims 1 to 5, in which X=CH₂, for flavoring a food product.

9. The use according to claim 8, **characterized in that** the compound of the formula (IVa) is the compound of the formula:

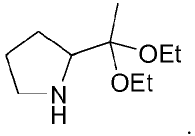

10. The use according to any one of claims 8 and 9, **characterized in that** the flavoring is obtained by forming a compound of the following formula (Ib): 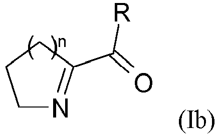
wherein R and n are as defined in claim 1,
from the compound of the formula (IVa).

11. The use according to claim 10, **characterized in that** the compound of the formula (Ib) is formed in a time-controlled manner.

12. A method for preparing a compound of the formula (IVa) as defined in claim 1, **characterized in that** it comprises the following series of steps:
(a2) protecting in the form of a ketal the exocyclic carbonyl function of a compound of the following formula (II) : 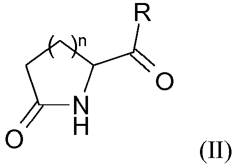 wherein R and n are as defined in claim 1,
to provide a compound of the following formula (IIIa) : 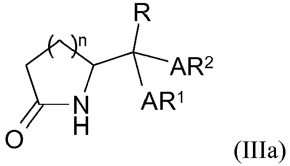 wherein n, A, R, R¹ and R² are as defined in claim 1,
(b2) reducing the compound of formula (IIIa) obtained from the previous step in the presence of hydride or deuteride ions, to provide a compound of the formula (IVa) as defined in claim 1.

13. The method according to claim 12, **characterized in that** the reduction of step (b2) is carried out in the presence of LiAlH₄ or LiAlD₄.

14. The method according to any one of claims 12 and 13, **characterized in that** the compound of the formula (II) is prepared from a compound of the following formula (VI) : 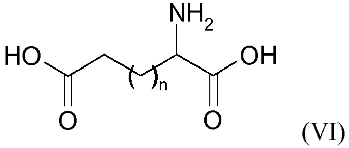
wherein n is as defined in claim 1,
by a Dakin-West reaction in the presence of an anhydride of the formula (RCO)₂O, where R is as defined in claim 1, followed by an alkaline treatment.

15. The method according to any one of claims 12 to 14, **characterized in that** the compound of the formula (IVa) is selected from:
